Europäisches Patentamt

European Patent Office

Office européen des brevets

(19)

(11) Veröffentlichungsnummer: **0 325 106 B1**

(12) **EUROPÄISCHE PATENTSCHRIFT**

(45) Veröffentlichungstag der Patentschrift: **21.10.92**

(51) Int. Cl.⁵: **G01N 33/84**

(21) Anmeldenummer: **89100101.8**

(22) Anmeldetag: **04.01.89**

(54) Verfahren und Test-Kit zur semiquantitativen Bestimmung des Kalziumgehaltes im Blut.

(30) Priorität: **05.01.88 DE 3800117**
**05.02.88 DE 3803463**

(43) Veröffentlichungstag der Anmeldung:
**26.07.89 Patentblatt 89/30**

(45) Bekanntmachung des Hinweises auf die
Patenterteilung:
**21.10.92 Patentblatt 92/43**

(84) Benannte Vertragsstaaten:
**AT BE CH DE ES FR GB IT LI NL SE**

(56) Entgegenhaltungen:
**EP-A- 0 204 045**
**DE-A- 2 147 066**
**FR-A- 2 425 642**

(73) Patentinhaber: **Linhart, Axel, Dr.**
**Rotterstrasse 46**
**W-8018 Grafing b. München(DE)**

(72) Erfinder: **Linhart, Axel, Dr.**
**Rotterstrasse 46**
**W-8018 Grafing b. München(DE)**

(74) Vertreter: **Zinnecker, Armin, Dipl.-Ing. Lorenz-**
**Seidler-Gossel et al**
**Widenmayerstrasse 23**
**W-8000 München 22(DE)**

**Beschreibung**

Die Erfindung betrifft ein verbessertes Verfahren zur semiquantitativen Schnellermittlung des Blut-Kalziumgehaltes sowie ein dazu geeignetes Test-Kit.

1. Bisherige Entwicklungen und Veröffentlichungen auf diesem Gebiet

Alle Testverfahren für eine semiquantitative Schnellermittlung des Blut-Kalziumgehaltes basieren auf der Beteiligung des Elementes Kalzium $Ca^{++}$ am Blutgerinnungsvorgang. Daher seien im Folgenden die Vorgänge bei der Blutgerinnung und ihre Beziehung zu $Ca^{++}$ kurz dargestellt.

Nach EDER (1976) läßt sich der Ablauf der Blutgerinnung wie folgt darstellen:
Beteiligt am Gerinnungsgeschehen sind sogenannte "plasmatische Faktoren" (im Blutplasma vorkommende Proteine oder Enzyme) und sogenannte "zelluläre Faktoren" (Phospholipide, die aus den Thrombozyten oder anderen Zellen stammen und wichtige Enzymreaktionen katalysieren).

Die Einleitung der Gerinnung kann auf zwei verschiedenen Wegen erfolgen. Sie kann erstens von einer Gewebsverletzung ausgehen und über das "exogene System" ablaufen (= extravasales, äußeres oder "extrinsic system"). Die zweite Möglichkeit stellt das sogenannte "endogene System" dar (= intravasales, inneres oder "intrinsic system"). Beide Systeme haben die Bildung eines Faktor-X-Aktivator-Komplexes als Ziel.

Das auslösende Moment im exogenen System ist eine Gewebsverletzung, wobei verschiedene gerinnungsaktive Substanzen, die unter dem Begriff **Thromboplastin** (Thrombokinase, Faktor III) zusammengefaßt werden, aus den alterierten Zellen frei werden.

Das endogene Reaktionssystem wird nach dem Kontakt des Blutes mit einer veränderten Oberfläche, wie sie z.B. bei Defekten oder Alterationen der Gefäßintima entsteht, in Gang gesetzt. Dabei wird der Hageman-Faktor (XII) aktiviert, der eine Reihe von Folgereaktionen auslöst, die schließlich den Faktor-X-Aktivatorkomplex bilden. Ab dem aktivierten Faktor Xa verlaufen nun exo- und endogenes System nach einem gemeinsamen Reaktionsmuster. Xa bewirkt zusammen mit $Ca^{++}$, Phospholipiden und dem Plasmafaktor V die Umwandlung von Prothrombin (Faktor II) zu **Thrombin (IIa).** Hilfsfaktoren hierfür nennt man Prothrombinaktivatoren.

Prothrombin ist ein Produkt der Leber, das sich normalerweise im Plasma befindet, Thrombin ist ein Spaltprodukt davon. **Thrombin bewirkt in seiner Eigenschaft als proteolytisches Enzym die Hauptreaktion des Gerinnungsvorganges, nämlich die Umwandlung des Fibrinogens (Faktor I) zu Fibrin.** Dabei werden vom Fibrinmolekül zwei Peptide abgespalten, es entstehen Fibrinmonomere, die sich zu Doppelsträngen polymerisieren und damit Fibrinfibrillen bilden.

Dieses Fibrin ist an sich noch löslich und wird erst nach Einwirkung des durch Thrombin und Kalzium aktivierten Faktor (VIII)(FSF) und durch weitere nichtkovalente $Ca^{++}$-Bindung zum kovalent vernetzten Fibrin. Das Blutgerinnsel ist entstanden. In diesem läuft später durch den Einfluß des Thrombozytenfaktors "Retraktoenzym" noch ein Schrumpfungsprozess der Fibrinfibrillen ab.

Aus dem Stand der Technik sind verschiedene Bestimmungsmethoden bekannt, die das Blutgerinnungsphänomen für den Kalziumnachweis nutzen. Das Prinzip besteht darin, daß durch Zugabe definierter Mengen an Ethylendiamintetraacetat (EDTA) Kalziumionen im Blut gebunden werden und somit nicht mehr für die Blutgerinnung zur Verfügung stehen. Werden dabei alle Kalziumionen chemisch gebunden, dann kann die Blutprobe nicht mehr gerinnen. Durch die Kenntnis der vorgelegten Menge an EDTA und des Befundes die Blutprobe ist geronnen oder nicht geronnen, ist festzustellen, ob der Kalziumgehalt im Blut einen vorgegebenen Grenzwert über- oder unterschreitet.

Semiquantitative Blut-Kalziumbestimmung beim Rind nach MAYER, RAGGI und RAMBERG (1965)

Der erste Schnelltest zur Kalziumbestimmung im Blut, der sich das Blutgerinnungsphänomen für den Kalziumnachweis zunutze machte, wurde 1965 von MAYER, RAGGI und RAMBERG entwickelt. Er stellt sozusagen die Grundlage für eine Reihe von modifizierten Testverfahren mit Vollblut dar.

Er wird von MAYER et.al. (1965) folgendermaßen beschrieben:
Die zur Verhinderung der Blutgerinnung benötigte Menge von Natriumsalz des Ethylendiamintetraacetat (EDTA) wird herangezogen, um die Kalziummenge des Blutes abzuschätzen. Es verbinden sich 0,01 ml der wässrigen Lösung von EDTA in einer bestimmten Konzentration 5 mEq./l mit 1 Mikrogramm Kalzium.

Für die Durchführung eines Testes werden 6 Blutröhrchen in der Größe 75 mm x 12 mm ∅ benötigt. In einer Tabelle werden die Mengen EDTA angegeben, die zur komplexen Bindung des Kalziums bei verschiedenen Kalziumkonzentrationen im Blut erforderlich sind. Zur Berechnung der erforderlichen EDTA-Menge wird ein mittlerer Hämatokrit von 33,3 % angenommen.

Für die Abfüllung der errechneten Menge an EDTA-Lösung in die Blutröhrchen wird eine exakte Mikrobürette benötigt. Jeder Test erfordert eine Serie von 6 Röhrchen, von denen 5 (entsprechend ihres zu erwartenden Serum-Kalziumgehaltes) mit der entsprechenden Menge Na-EDTA-lösung (siehe Tabelle) in einer Konzentration von 5 mEq./l beschickt werden. Das 6. Blutröhrchen - ohne EDTA - dient als Vergleich zur Feststellung der Blutgerinnungsfähigkeit.

Die Röhrchen werden wie folgt bezeichnet:

```
Röhrchen        ohne      EDTA-Lösung 0
   "      mit 0,40 ml        "        6 = 6 mg/dl
   "      mit 0,47 ml        "        7 = 7 mg/dl
   "      mit 0,53 ml        "        8 = 8 mg/dl
   "      mit 0,60 ml        "        9 = 9 mg/dl
   "      mit 0,67 ml        "       10 =10 mg/dl
```

Nach Einbringen der EDTA-Lösung wird das Wasser durch Erhitzen auf 105° C verdampft, und alle 6 Röhrchen jeder Serie werden mit einem durchstechbaren Gummistopfen verschlossen. Vor Durchführung der Serum-Kalziumbestimmung mit diesem Test wird eine dünne Kanüle in den Gummistopfen eingestochen, um den Druckausgleich beim Einspritzen des Blutes in die Blutröhrchen zu gewährleisten. Die Blutprobe wird mit einer Plastikspritze (5 ml) unter Verwendung feiner Kanülen entnommen und jeweils exakt 1,0 ml Blut in die Röhrchen 6 bis 10 abgefüllt. Die Röhrchenserie wird anschließend in ein Wasserbad von 37° C gestellt.

Die Beurteilung des Testes ist nach etwa 15 Minuten möglich. Bei Raumtemperatur dauert es laut Angabe von MAYER et.al. (1965) bis zu zwei Stunden, bis das Ergebnis ablesbar ist.

Eine Blutprobe gilt als geronnen, wenn sich das Blut bei leichtem Schwenken des umgekehrten Röhrchens nicht vom Röhrchenboden löst.

Der Kalziumgehalt der Blutprobe wird wie folgt abgelesen:

```
Gerinnung                                Serum-Kalziumgehalt
Gerinnung nur in Röhrchen 0              6 mg/dl
   "      "   "     "      0,6           6 mg/dl u.  7 mg/dl
   "      "   "     "      0,6,7,        7 mg/dl u.  8 mg/dl
   "      "   "     "      0,6,7,8       8 mg/dl u.  9 mg/dl
   "      "   "     "      0,6,7,8,9     9 mg/dl u. 10 mg/dl
   "          "     "      0,6,7,8,9,10 10 mg/dl
```

Das in den Röhrchen eventuell noch im "Überschuß" befindliche Kalzium läßt einen Blutgerinnungsvorgang gemäß dem endogenen Blutgerinnungssystem ablaufen.

Durch die Testreihe mit verschieden hohen EDTA-Mengen läßt sich der Blut-Kalziumwert in einzelnen Stufen gut abschätzen. Die Testtemperatur wurde auf 37° C festgelegt, um im gewählten Zeitraum von 15 Minuten vergleichbare und reproduzierbare Ergebnisse zu erzielen.

HARRY (1973) kam nach der Durchführung des Tests in der oben beschriebenen Weise zu folgendem Ergebnis:

Tab. 1

| Vergleich der mit dem EDTA-Test ermittelten und der atomabsorptionsspektrometrisch festgestellten Serum-Kalziumgehalte bei 144 klinisch gesunden und 36 festliegenden Kühen | | | |
|---|---|---|---|
| | klinisch gesunde Kühe | | festliegende Kühe |
| | Röhrchen geschwenkt | Röhrchen unbewegt | |
| Übereinstimmung | 2 (12,5%) | 71 (55,5 %) | 13 (36,1 %) |
| Abweichung | 14 (87,5%) | 57 (44,5 %) | 23 (63.9 %) |

Tab. 2: Beurteilung des Kalziumgehaltes im Blut von 180 Rindern mit dem semiquantitativen EDTA-Test in Bezug zu den Ergebnissen der quantitativen, atomabsorptionsspektrometrischen Kontrolluntersuchungen gemäß den Untersuchungsergebnissen von HARRY (1973):

| | | Ergebnis der atomabsorptionsspektrometrischen Analyse | | |
|---|---|---|---|---|
| Ergebnis des semiquantit. EDTA-Tests | | Ca normal und ↑ | Ca ↓ | |
| | Ca normal und ↑ | 81 | 18 | 99 |
| | Ca ↓ | 76 | 5 | 81 |
| | | 157 | 23 | 180 |

Von 23 hypokalzämischen Tieren klassifizierte der Test 5 richtig. Demnach hat der Test eine "Empfindlichkeit" von 22 %.

Von 157 nicht hypokalzämischen Tieren wurden 81 vom EDTA-Test richtig ausgewiesen. Die "Spezifität" des Testes liegt bei 52 %.

Die "Richtigkeit" des Testes ist 48 %, da er 86 von 180 Probanden insgesamt richtig klassifizierte. Von 81 Tieren, die der EDTA-Test als hypokalzämisch auswies, hatten nur 5 tatsächlich hypokalzämische Blutwerte. Bei diesem "Prädiktiven Wert des positiven Tests" (VECCHIO, 1966), genannten Kriterium erreichte der Test nur einen Wert von 6 % (pvT+).

Der "Prädiktive Wert des negativen Tests" (pvT-) erreichte einen Wert von 82 %, da von 99 als nicht hypokalzämisch eingestuften Tieren 81 tatsächlich nicht hypokalzämisch waren.

Um die Aussagekraft des "Prädiktiven Wertes" (pvT+) abschätzen zu können, ist es wichtig, die sogenannte "Prävalenz" der an Hypokalzämie erkrankten Tiere in der untersuchten Gruppe zu kennen. Die "Prävalenz" beträgt in diesem Fall 13 %. Dieser Wert beschreibt, daß 23 von 180 Tieren hypokalzämisch waren.

Da gerade die erniedrigten Serum-Kalziumwerte diagnostisch wichtig sind, kam HARRY (1973) zu dem Schluß, der Mayer-EDTA-Test habe **keinen** praktisch-diagnostischen Wert.

MARTIG et.al. (1974) führten den Mayer-EDTA-Test ebenfalls in vorgegebener Form bei 50 Kühen durch und erhielten die in Tabelle 3 zusammengefaßten Ergebnisse:

Tab. 3: Vergleich der mit dem semiquantitativen EDTA-Test festgestellten Kalziumgehalte im Blut mit den Ergebnissen der quantitativen atomabsorptions-spektrometrischen Kontrolluntersuchungen:

| Ergebnis des EDTA-Tests | | Ergebnis der atomabsorptions-spektrometrischen Analyse | | |
|---|---|---|---|---|
| | | Ca normal und ↑ | Ca ↓ | |
| | Ca normal und ↑ | 30 | 1 | 31 |
| | Ca ↓ | 0 | 19 | 19 |
| | | 30 | 20 | 50 |

| - Testempfindlichkeit: | 95 % |
|---|---|
| - Testspezifität: | 100 % |
| - Testrichtigkeit: | 98 % |
| -pvT + | 100 % |
| -pvT- | 97 % |
| - Prävalenz: | 40 % |

REITZ (1978) wählte die Versuchsbedingungen so einfach und praxisnahe wie möglich, entnahm 244 Blutproben mittels einer Recordspritze mit Recordkanülen und schwenkte die Probe zur besseren Lösung des EDTA am Anfang des Versuchs leicht. Als Versuchstemperatur wählte er die herrschende Umwelttemperatur und führte die Beurteilung der Proben dementsprechend erst nach 1 - 2 Stunden durch. In dieser Zeit wurden die Proben innerhalb von 1/2 - 1 Stunde im Auto zu seinem Labor überbracht und jeweils eine flammenphotometrische Gegenprobe gemacht. Von den 244 Proben konnten 25 nicht ausgewertet werden.

Tab. 4: Vergleich von 219 mit dem Mayer-EDTA-Test ermittelten Kalziumwerten mit den flammenphotometrisch festgestellten Serum-Kalziumwerten gemäß den Untersuchungsergebnissen von REITZ (1978):

| Ergebnis des EDTA-Tests | | Ergebnis der flammenphotometrischen Analyse | | |
|---|---|---|---|---|
| | | Ca über 7 mg/dl | Ca unter 7 mg/dl | |
| | Ca über 7 mg/dl | 79 | 18 | 97 |
| | Ca unter 7 mg/dl | 47 | 75 | 122 |
| | | 126 | 93 | 219 |

| - Testempfindlichkeit: | 81 % |
|---|---|
| - Testspezifität: | 63 % |
| - Testrichtigkeit: | 70 % |
| - pvT + | 61 % |
| -pvT- | 81 % |
| - Prävalenz: | 42 % |

Semiquantitative Blut-Kalziumbestimmung beim Rind nach SANDHOLM, JÖNSSON, PEHRSON und ZEWI (1979)

SANDHOLM et.al. (1979) beschrieben einen modifizierten EDTA-Test bei dem das Blutkalzium durch ein Überangebot von $K_2$-EDTA komplex abgebunden wird und der EDTA-Überschuß dann mit $Ca^{++}$ und Thromboplastin als Reaktionsbeschleuniger "rücktitriert" wird. Es wird hierbei eine künstliche Aktivierung des sehr schnell ablaufenden exogenen Blutgerinnungsweges erzielt. Damit soll der Test innerhalb eines angemessenen Zeitraums ( 5 Minuten) bis zum Endpunkt der Gerinnung geführt werden, um so Unsicherheitsquellen und Fehler in der Tesbeurteilung zu reduzieren.

Im Folgenden ist die praktische Ausführung dieses Testes kurz beschrieben:
- Reagenzröhrchen "1":
  In ein Kunststoffröhrchen mit 11 mm Durchmesser und einer Markierung bei 10 ml werden jeweils 1 ml Antikoagulationslösung (10,1 mg $K_2$-EDTA = 25,0 $\mu$Mol) verbracht und anschließend eingetrocknet.
- Reaktionsampullen "2":
  In diese kommen jeweils 1 ml Thromboplastinsuspension und 11,7 $\mu$Mol $CaCl_2$. Der Inhalt wird gefriergetrocknet und unter Stickstoff verschlossen.

1. Das Reagenzröhrchen "1" wird mit Nativblut bis zur Markierung gefüllt. Das Röhrchen wird 20mal gekippt, um den Inhalt gut zu durchmischen (nicht schütteln). Dabei wird das EDTA in Röhrchen "1" das Kalzium des Blutes binden und eine Blutgerinnung verhindern.

2. Die Reaktionsampulle "2" wird geöffnet, und etwa 10 Tropfen destilliertes Wasser aus dem Plastiktropfer werden zugefügt. Durch leichtes Schütteln wird das Pulver im Fläschchen gelöst.

3. Der Inhalt des Reagenzröhrchens "1" wird zusammen mit dem der Ampulle "2" in ein weiteres Reagenzglas überführt, dieses dann verschlossen und der Inhalt durch 20maliges Kippen vermischt. Dann das Fläschchen stehenlassen. Jetzt wird das Kalzium das überschüssige EDTA zurücktitrieren.

Bleiben daraufhin freie Ca-Ionen in der Probe, werden diese mithilfe des Thromboplastins die Blutgerinnung einleiten. Wurden dagegen alle Kalzium-Ionen mit EDTA gebunden, kann die Blutgerinnung nicht ablaufen. Die Reaktionszeit beträgt 5 Minuten.

Der Test wird bei Zimmertemperatur ausgeführt, niedrigere Temperaturen beeinflussen die Reaktionszeit negativ.

Der Test ist auf den Grenzwert von 2,0 mMol Kalzium pro dl Serum ausgelegt (= 8 mg/dl), da dieser als unterste Grenze für den physiologischen Blut-Kalziumwert von Kühen angesehen wird (SANDHOLM et.al., 1979).

Auswertung:

Die Ablesung erfolgt nach 5 Minuten
- Probe koaguliert: Der Kalziumgehalt der Probe liegt über dem gewählten Grenzwert von 8 mg/dl.
- Probe nicht koaguliert: Der Kalziumgehalt der Probe liegt unter 8 mg/dl = Hypokalzämie.
- Probe an der Grenze zur Koagulation: (Schlieren und kleine Koagula werden sichtbar.) Der Kalziumgehalt der Probe liegt sehr nahe am Grenzwert.

Von allen Probanden wurden parallel Blutproben mittels eines Atomabsorptionsspektrophotometers auf ihren Kalzium- und Magnesiumgehalt untersucht und zudem der Hämatokritwert ermittelt.

Tab.5: Vergleich der Ergebnisse von 158 mit dem EDTA-
Test nach SANDHOLM et al. (1979) durchgeführten
semiquantitativen Kalziumbestimmungen mit denen der
quantitativen atomabsorptionsspektrometrischen
Untersuchung

| | | Ergebnis der atomabsorptions-spektrometrischen Analyse | | |
|---|---|---|---|---|
| | | Ca über dem untersuchten Grenzwert | Ca unter dem untersuchten Grenzwert | |
| Ergebnis des EDTA-Tests | geronnen | 58 | 11 | 69 |
| | nicht geronnen | 4 | 85 | 89 |
| | | 62 | 96 | 158 |

| | |
|---|---|
| - Testempfindlichkeit: | 89 % |
| - Testspezifität: | 94 % |
| - Testrichtigkeit: | 91 % |
| -pvT + | 96 % |
| -pvT- | 84 % |
| - Prävalenz: | 56 % |

Schnelltest zur semiquantitativen Bestimmung des Blut-Kalziumgehaltes beim Rind nach LINHART (1984)

Entwickelt von Axel Linhart im Rahmen einer Inaugural-Dissertation zur Erlangung der tiermedizinischen Doktorwürde an der tierärztlichen Fakultät München unter der Leitung von Prof.Dr.Dr.h.c. G. Dirksen (1984).

Funktionsprinzip:

Die $Na_2$-EDTA-Vorlage im Teströhrchen ist so berechnet, daß 0,466 mg Kalzium (das ist die absolute Menge Gesamtkalzium, die im Serum von 10 ml Vollblut enthalten ist, wenn der Hämatokrit 33,5 Vol. % und die Kalziumkonzentration im Serum 7 mg/dl beträgt) komplex abgebunden werden können. Dieses Kalzium steht dann nicht mehr für die Blutgerinnung zur Verfügung. Sind nun im Serum mehr als 7 mg/dl Kalzium vorhanden, so kann der exogene Blutgerinnungsweg noch ablaufen, und das Testblut gerinnt innerhalb von 5 Minuten nach Zugabe des Thromboplastins.

Sind jedoch weniger als 7 mg Kalzium pro dl vorhanden, so ist praktisch das gesamte Kalzium durch EDTA abgebunden, und eine Gerinnung des Testblutes ist damit unmöglich. Aus dem Testergebnis "geronnen/ nicht geronnen" ist auf diese Weise eine Aussage darüber möglich, ob der Blut-Kalziumgehalt des Tieres unter dem Grenzwert von 7 mg/dl abgesunken ist oder nicht.

Da der Reaktionsablauf bei einer geringen Restmenge von nicht an EDTA gebundenem Kalzium sehr langsam ist und bis zu 2 Stunde dauern kann (MAYER et al., 1965), wird das Thromboplastin als Reaktionsbeschleuniger eingesetzt. Mit der ermittelten Thromboplastinmenge ist es möglich, im positiven Fall den Koagulationsendpunkt und damit das Testergebnis schon innerhalb 5 Minuten zu erreichen.

Auswertung der Reaktion:

5 Minuten nach Ansetzen des Testes wird die Reaktion wie folgt beurteilt:
- Probe koaguliert:
  Der Kalziumgehalt der Probe liegt über dem gewählten Grenzwert von 7 mg/dl.

- Probe nicht koaguliert:
  Der Kalziumgehalt der Probe liegt unter 7 mg/dl.
- Probe an der Grenze zur Koagulation:
  (Schlieren und kleine Koagula sind sichtbar, beim Kippen des Röhrchens fließt das Blut.) Der Kalziumgehalt der Probe liegt nahe am Grenzwert.

Ausführung des Kalziumtestes:

- Nachdem einige Tropfen Blut aus der, in die Jugularvene eingeführten Kanüle abgeflossen sind, wird die Spritze bis über die 10,0 ml-Marke hinaus mit Blut abgefüllt und davon anschließend soviel verworfen, bis sich ein blasenfreier Inhalt von 10,0 ml ergibt.
- Im Rahmen der eigenen Versuche wurden außerdem noch je ein Reagenzröhrchen aus Kunststoff zur quantitativen Bestimmung des Kalziumgehaltes mittels der flammenphotometrischen Referenzmethode sowie zur Ermittlung des Hämatokritwertes mit Blut gefüllt.
- Die 10,0 ml Blut werden nun in das Reagenzröhrchen mit der $Na_2$-EDTA-Vorlage verbracht, dieses sofort verschlossen und zirka 10mal geschwenkt (nicht geschüttelt), um den Inhalt gut zu durchmischen.
- 0,1 ml der Thromboplastinlösung wird mit einer 100 Mikroliterpipette zusätzlich einpipettiert, und das Röhrchen wiederum etwa 10mal zum Durchmischen geschwenkt.
- Jede volle Minute wird durch Kippen überprüft, ob eine Koagulation eingetreten ist.
- Testende nach 5 Minuten.

Tab. 6: Vergleich der Ergebnisse von 104 mit dem EDTA-Test durchgeführten semiquantitativen Kalziumbestimmungen mit denen der quantitativen flammenphotometrischen Untersuchung

| | | Ergebnis der quantitiven flammenphotometrischen Analyse | | |
| --- | --- | --- | --- | --- |
| | | Ca über 7 mg/dl | Ca unter 7 mg/dl | |
| Ergebnis des semiquan. EDTA-Tests | Ca über 7 mg/dl | 57 | 11 | 68 |
| | Ca unter 7 mg/dl | 3 | 33 | 36 |
| | | 60 | 44 | 104 |

| | |
| --- | --- |
| - Testempfindlichkeit: | 75 % |
| - Testspezifität: | 95 % |
| - Testrichtigkeit: | 87 % |
| - pvT + | 92 % |
| -pvT- | 84 % |
| -Prävalenz: | 42 % |

"Ca-Test Graeub" (Rehagstr. 83, CH-3018 Bern; Vertrieb Deutschland: Fa. Albrecht, Aulendorf)

Der 1985 veröffentlichte Reaktionsablauf dieser Testanordnung entspricht dem chemischen Reaktionsprinzip des von SANDHOLM et al. (1979) beschriebenen Ca-Testes.

Es wird also das Blutkalzium durch ein Überangebot von EDTA komplex abgebunden und der EDTA-Überschuß anschließend mit $Ca^{++}$ und Thromboplastin als Reaktionsbeschleuniger "rücktitriert". Dazu befindet sich eingedampftes EDTA am Röhrchenboden und ein nicht näher beschriebenes Gemisch aus Thromboplastin und $CaCl_2$ in einem eindrückbaren Stöpselmechanismus. Dieses "Gemisch" wird durch Eindrücken einer Stöpseltrennwand der Blutmenge beigemischt, die entsprechend dem gewünschten Testwert (5, 7, 8 mg/dl) vorher durch Schwenken des Röhrchens mit dem EDTA-Überschuß vermischt worden war. Anschließend wird das Röhrchen 1/2 Minute zur Durchmischung der Reagenzien geschwenkt, 5 Minuten stehen gelassen und dann das Testergebnis wie vorher beschrieben abgelesen. Das Testurteil geronnen / nicht geronnen wird bezüglich des gewählten Testwertes interpretiert.

Bewertungen des Ca-Test-Graeub sollen im Rahmen einer Doktorarbeit an der Tierärztlichen Fakultät Bern erstellt worden sein. Sie konnten leider bisher noch nicht eingesehen werden.

Bislang sind dem Großtierpraktiker vor Ort und fernab des eigenen Praxislabors zur raschen Beurteilung des Kalzium-Blutwertes des Patienten und als Entscheidungshilfe für die Wahl der richtigen Therapie nur die vorab beschriebenen semiquantitativen Ca-Schnelltests zur Wahl gestellt.

Auf dem Bereich der "Trockenchemie" ist von der Firma Boehringer Mannheim ein mehrschichtiger Teststreifen zur Blut-Ca-Bestimmung angekündigt, der colorimetrisch mittels eines transportablen Reflektionsphotometers ausgewertet werden kann. Nachteil dieser Methode ist, daß hierfür erst das teure Gerät zur Bestimmung angeschafft werden muß und stets im Auto mitgeführt werden muß, was durch Verschmutzung und Erschütterung im Fahrzeug zur vorschnellen Alterung des Präzisionsgerätes beiträgt.

**2. Die zum Schutz angemeldete Neuentwicklung nach Dr. Axel Linhart**

Dieser Kalzium-Schnelltest basiert auf den Erkenntnissen der unter 1. beschriebenen bisherigen Entwicklungen von semiquantitativen Kalzium-Schnelltests.

Die Idee wurde im Juli 1982 gefunden, als ich mich mit der vorgegebenen Dissertationsarbeit beschäftigte. In der Folgezeit wurde dann diese Idee in vielen Entwicklungsstufen weiterverfolgt und immer wieder unter Praxisverhältnissen zum Einsatz gebracht. Zur Zeit liegen über 400 ausgewertete Testansätze zur Beurteilung bereit. Entwicklungsziel war, die bekannten Nachteile der bisherigen semiquantitativen Ca-Schnelltests (incl. des mittlererweile dazugekommenen "Ca-Test-Graeub") abzubauen bzw. zu vermeiden und den Testvorgang nach folgenden Kriterien zu gestalten:
- Einfache und billige Materialien,
- unkomplizierter Reaktionsablauf,
- lange Haltbarkeit der Reagenzien und des Testansatzes,
- einfache Handhabung,
- sicheres Erreichen des Testendpunktes innerhalb von 5 Minuten,
- möglichst große Genauigkeit und Verläßlichkeit der der Testaussage.

**3. Nachteile der bis dahin vorgestellten semiquantitativen Blut-Kalziumbestimmungsmethoden.**

3.1. Der Test nach MAYER et al. (1965)

Nachteile bei diesem Testansatz sind vor allem die geringe Testmenge des Blutes von 1 ml und das Fehlen einer zufügbaren Substanz, die selbst bei geringstem Überschuß an Kalzium zuverlässig und unabhängig von der Umgebungstemperatur den Reaktionsendpunkt und damit die Testaussage innerhalb von 5 Minuten erreichen läßt.
0,1 ml Einfüllmengenfehler bedeuten bei 1 ml Testblutmenge einen Fehler von 10 %, bei 10 ml Testblutmenge aber nur einen Aussagefehler von 1 % bezüglich des Testgrenzwertes.

3.2. Der Test nach SANDHOLM et al. (1979)

Der von SANDHOLM et al. (1979) beschriebene Kalzium-Schnelltest wurde von der Fa. Orion Diagnostica, Helsinki auf dem Markt eingeführt, aber kurze Zeit später die Produktion wieder eingestellt.
Der Nachteil dieser Testanordnung unter Praxisverhältnissen besteht darin, daß 3 Reaktionsgefäße, 1 sterile Nadel und Spritze und 1 Pipettenröhrchen mit destilliertem Wasser verwendet werden müssen,um letztendlich eine Testaussage zu erhalten. Die Handhabung ist dadurch fehlerträchtig und umständlich, läßt somit von einer häufigen Verwendung Abstand nehmen.
Produktionstechnisch besteht eine Schwierigkeit darin, daß pro Produktionsreihe eine neue Eichkurve für das Thromboplastin nötig ist, da die "Aktivität" von Thromboplastin nicht "standardisiert" ist und somit die benötigte Thromboplastinmenge für den Testansatz immer wieder neu bestimmt werden muß.

Ein entscheidender Nachteil dieses Tests liegt darin, daß die Aktivität des Thromboplastins praktisch für jeden Versuch neu bestimmt werden muß, da die Aktivität von Thromboplastin nicht standardisierbar ist.

### 3.3. Der Test nach LINHART (1984)

Für den semiquantitativen Kalzium-Schnelltest, der von mir im Rahmen der Dissertationsarbeit entwikkelt wurde gilt, daß die vorab beschriebenen Nachteile der Testanordnungen so weit wie damals möglich beseitigt wurden. Aber auch hier steht einer Serienreife entgegen, daß das Thromboplastin in seinem "Aktivitätsgehalt" nicht standardisierbar ist, also seine benötigte Menge für den Testansatz immer wieder neu bestimmt werden muß. In flüssiger Form ist das Thromboplastin außerdem nur für kurze Zeit bei Raumtemperatur haltbar, muß also zur Haltbarmachung lyophilisiert werden.

Für die Handhabung beim Testvorgang bedeutet dies wiederum den Nachteil, daß entweder 2 Röhrchen hintereinander mittels Umfüllung des Testblutes verwendet werden müssen, oder daß ein ähnlich komplizierter wie anfälliger und fehlerträchtiger Stöpselmechanismus wie beim Ca-Test-Graeub zur Anwendung kommen muß, der das Thromboplastin erst frei gibt, wenn sich das Testblut mit der EDTA-Vorgabe vermengt hat.

### 3.4. Der Ca-Test-Graeub

Beim Ca-Test-Graeub wiederum wurde der Versuch gemacht, die Vorteile des Ca-Schnelltestes nach SANDHOLM et al. (1979) mit denen der, in der Dissertationsarbeit nach LINHART (1984) veröffentlichten, zu vereinen.

Wieder einmal zeigen sich seine Nachteile erst bei der Anwendung unter Praxisverhältnissen:

- Das Röhrchen ist zu dick für die herkömmlichen Reagenzglasständer.
- 3 mögliche Einfüllmengen bzw. Testwerte verwirren und führen schnell zu falscher Einfüllmenge.
- Der Stöpselmechanismus ist teuer, schwer einzudrücken und öffnet sich oft nur unzureichend, wodurch eine vollständige Vermengung des Ca-Thromboplastingemenges mit dem Blut erschwert wird. Verfälschte Testaussagen (± 2 mg/dl Blut-Ca; gemäß eigenen Untersuchungen) sind hierdurch und durch die Tatsache möglich, daß bis zu 1,5 ml in den Ritzen und Ecken des Stöpselmechanismus durch Adhäsion und Spontangerinnung zurückbleiben können.

Trotz zahlreicher Bemühungen steht damit dem Großtierpraktiker vor Ort und fernab des eigenen Praxislabors keine voll befriedigende Bestimmungsmethode zur Beurteilung des Kalzium-Blutwertes als Entscheidungshilfe für die Wahl der richtigen Therapie zur Verfügung. Die bekannten Testmethoden sind zu ungenau, erfordern Reaktionsbedingungen, die in den meisten Fällen vor Ort nicht vorhanden sind oder sind in ihrer Durchführung zu komplex, so daß sehr leicht "Fehlbestimmungen" erfolgen können. Außerdem ist das als Reaktionsbeschleuniger eingesetzte Thromboplastin nur unter besonderen Bedingungen längere Zeit haltbar.

Aus der EP 215 735 A1 ist ein veterinärmedizinischer Reagenziensatz in Teströhrchen und dazugehörendem Stopfen zur Bestimmung des Kalziumgehaltes im Blut bekannt, bei dem die abgemessene Menge Blut mit einer vorbestimmten Menge Komplexbildner und anschließend einer vorbestimmten Menge Kalzium-Ionen versetzt wird, so daß, entsprechend dem Kalziumgehalt des Testblutes, eine vollständige, teilweise oder keine Koagulation stattfindet. Der Komplexbildner liegt in einem Teströhrchen, welches zur Blutaufnahme dient, vor. Vorbestimmte Mengen eines Kalziumsalzes und ein Koagualationsbeschleuniger sind in einem Hohlraum des zum Teströhrchen gehörenden Stopfens vorhanden, wobei der stopfen so ausgebildet ist, daß sein Inhalt in das mit dem Stopfen verschlossene Röhrchen freigegeben werden kann. Als Gerinnungsbeschleuniger wird Thromboplastin verwendet.

Aus der DE-OS 26 12 719 ist ein Verfahren zur Ermittlung der Serumkoagulationsfähigkeit bei Patienten bekannt. Dieses Verfahren dient zum Thrombose- und Präthrombosenachweis. Der Vorveröffentlichung liegt die Aufgabe zugrunde, eine rasch durchzuführende und zuverlässige Maßnahme zur Ermittlung des Zustandes eines Individuums hinsichtlich der Gerinnungstendenz seines Blutes zu schaffen. Hierdurch soll ermittelt werden, ob das Individuum im Hinblick auf eine bevorstehende Koronartherienerkrankung einer Notbehandlung bedarf. Es soll also ein Diagnosetest geschaffen werden, der die Koagulationsfähigkeit des Blutes ermitteln kann.

Aus der EP-A-204 045 ist es bekannt, Reagenzien bei Blutplasmagerinnungstests zu verwenden, die entweder zu medizinischen Diagnosezwecken oder zur Überwachung von gewissen Arzneimitteltherapien mit Antikoagulierungsmitteln durchgeführt werden. Die Reagenzien können Thrombin oder Thromboplastin enthalten. Das aktive Reagenz Rinderthrombin wird mit einem Puffer, mit Saline, mit einem bakterienwachstumhemmenden Schutzmittel und mit Rinderserumalbumin vermischt, um ein Reagenz zu bilden. Diese Lösung wird auf einen pH-Wert von etwa 8,0 eingestellt und dann in großen Mengen gefriergetrocknet. Das gefriergetrocknete Reagenz wird mit einem Bindemittel und einem Schmiermittel trocken vermengt, um eine Mischung herzustellen, die dann verdichtet, granuliert und tablettiert werden kann. Das Schmiermittel kann allerdings auch nach dem Granulieren hinzugefügt werden.

Aus der EP-A-204 045 ist es demnach bekannt, ein Blutplasmagerinnungsreagenz in mikrokonzentrierter Tablettenform durch das trockene Vermischen eines Bindemittels und eines Schmiermittels mit einem oder mehreren gefriergetrockneten aktiven Bestandteilen und mit einem Puffer herzustellen. Das trocken vermischte Gemisch wird verdichtet, granuliert und tablettiert.

Aufgabe der Erfindung ist es, einen verbesserten semiquantitativen Kalzium-Schnelltest vorzuschlagen, der "vor Ort" eine einfache, schnelle und zuverlässige Kalziumbestimmung im Blut ermöglicht.

Erfindungsgemäß wird diese Aufgabe durch das im Anspruch 1 angegebene Verfahren gelöst. Die Kalzium-Ionen in einer Blutprobe werden entsprechend eines vorgegebenen Kalziumgrenzwertes mit einer definierten Menge eines Komplexbildners ganz oder teilweise komplexiert. Anschließend wird die eventuell einsetzende Gerinnung durch einen Gerinnungsbeschleuniger aktiviert. Der Gerinnungsbeschleuniger ist Thrombin.

Erfindungsgemäß wird also vorgeschlagen, den Kalziumtest so durchzuführen, daß eine exakt definierte Menge eines Komplexbildners vorgelegt wird, das Blut zugegeben und die eventuell eintretende Gerinnung des Blutes durch Thrombin beschleunigt wird.

Die erfindungsgemäße Verwendung von Thrombin weist den Vorteil auf, daß Thrombin mit standardisierter Aktivität im Handel als Pulver erhältlich ist, während das bislang verwendete Thromboplastin in seinem Aktivitätsgehalt nicht standardisierbar ist und für jeden Testansatz neu bestimmt werden müßte. In der DE-OS 18 04 292 und der EP 241 613 A1 wird zwar angeblich standardisiertes Thromboplastin erwähnt. Tatsächlich existiert jedoch bis heute keine Methodik der vergleichenden Aktivitätsmessung für Thromboplastin und somit auch keine international anerkannte Maßeinheit für die biologische Aktivität von Thromboplastinextrakten. Es wird in der Praxis davon ausgegangen, daß man bei der Zellextraktion aus gleichen Körperzellen immer ungefähr gleichviel Thromboplastin gewinnen kann. Dieser Ausgangspunkt ist jedoch nicht in einem Maß zutreffend, daß von einem in seinem Aktivitätsgehalt standardisierbaren Thromboplastin gesprochen werden könnte. Auch die beiden genannten Vorveröffentlichungen setzen ein standardisiertes Thromboplastin lediglich voraus, ohne ein Verfahren anzugeben, wie eine standardisierte Thromboplastin-Tablette hergestellt werden könnte.

Als Komplexbildner sind alle bekannten Chelatbildner geeignet, die die freien Kalzium-Ionen im Blut komplexieren können, ohne dabei jedoch die Gerinnung des Blutes zu beeinflussen. Geeignete Verbindungen sind z.B. Ethylendiamintetraacetat (EDTA) sowie dessen Natrium- und Kaliumsalze.

Der semiquantitative Schnelltest kann beispielsweise wie folgt durchgeführt werden:
Eine vorgegebene Menge des Komplexbildners wird mit dem Blut vermischt und kurze Zeit später mit dem Thrombin als Gerinnungsbeschleuniger versetzt. Durch die Vorgabe einer definierten Menge an Komplexbildner wird ein Grenzwert festgesetzt, bis zu dem alle vorhandenen Kalzium-Ionen im Blut komplexiert werden. Auch nach der Zugabe von Thrombin erfolgt innerhalb der Testzeit keine Gerinnung des Blutes. Wird dieser Grenzwert überschritten, so bewirken die nicht komplexierten Kalzium-Ionen in Gegenwart von Thrombin eine Blutgerinnung innerhalb von 5 Minuten. Die Grenzwerte können in Abhängigkeit der Spezies und der Indikationsstellung durch den Gehalt an Komplexbildner variiert werden. Für einen Kalziumgrenzwert von 7 mg/dl sind 4,313 mg Na2-EDTA und ca. 2 bis 8 i.E. Thrombin einzusetzen, wenn der mittlere Hämatokrit 33,5 % beträgt.

Die Reaktionsgeschwindigkeit des Tests hängt ab von der Umwelttemperatur, der Menge der nach der Reaktion mit Na2-EDTA noch freien Ca-Ionen und der Menge des zugefügten, katalytisch wirkenden Thrombins.

Der Einfluß der Umwelttemperatur ist im Bereich zwischen 10° C und 40° C sehr gering; Testproben gleichen Gehaltes an Ca und Thrombin erreichten ihren Reaktionsendpunkt bei 40° C nur wenige Sekunden schneller als bei 10° C.

Mindestens zwei i.E. Thrombin waren nötig, um auch bei Proben mit einem Ca-Gehalt im engsten Bereich um den eingestellten Testwert herum noch innerhalb der angestrebten 5 Minuten das Testende und damit die Testaussage sicher zu erreichen.

3 i.E. Thrombin stellen voll das Idealmaß dar.

Aber auch bis zu 8 i.E. Thrombin verfälschten das Testergebnis nicht, lediglich das Testende wurde damit schneller erreicht. Mehr als 8 i.E. Thrombin führen zum überstürzten katalytischen Einsetzen der Blutgerinnung, unter Umständen auch schon bevor die Komplexbildung von Na2-EDTA ganz abgeschlossen ist. Als Resultat ergäben sich verfälschte Testaussagen.

Tatsächlich konnte festgestellt werden, daß geringste Mengen von "überschüssigen" Ca-Ionen ausreichen, um eine Gerinnung der Probe zu ermöglichen. So gerannen Proben mit 7,1 mg/dl, ja sogar mit 7,05 mg/dl (Voraussetzung: genaueste Einwaagen von Na2-EDTA und 10 ml Blut) noch ganz klar und in deutlicher Unterscheidung zu Proben mit einem Testwert von unter 7 mg/dl.

Die Reaktionsgeschwindigkeit des Tests korrelierte nur bei Zugabe von weniger als 2 i.E. Thrombin mit der Menge des ungebundenen Kalziums; über 2 i.E. Thrombin war dieses Phänomen von untergeordneter, nicht meßbarer Bedeutung.

Das in das Teströhrchen eingebrachte, in dem Röhrcheninneren großflächig anhaftende Na2-EDTA ist spätestens eine halbe Minute nach dem Einfüllen und dem ersten Schwenken des Röhrchens vollständig gelöst und komplexiert.

In einer bevorzugten Ausführungsform ist das erfindungsgemäße Verfahren in einem einfach handzuhabenden Test-Kit zusammengefaßt. Der Test-Kit besteht aus einem verschließbaren Röhrchen (z.B. ein Reagenzglas) aus Glas oder Kunststoff. In diesem Röhrchen wird die gewünschte Menge des Komplexbildners vorgelegt. Dies kann z.B. dadurch erfolgen, daß eine entsprechend molare Lösung des Komplexbildners in dem Röhrchen vorgelegt und eingedampft wird oder aber der Komplexbildner in Form einer leicht löslichen Tablette oder Kapsel eingebracht wird. Dem Fachmann sind Verfahren zur Herstellung solcher Tabletten oder Kapseln hinreichend bekannt, so daß dies nicht näher erläutert werden muß.

Zusätzlich zu dem Komplexbildner wird eine weitere Tablette in dem Röhrchen vorgelegt, die aktives Thrombin enthält. Wesentlich ist hierbei, daß sich die Thrombin enthaltende Tablette erst dann löst, wenn die Komplexbildung zwischen dem Komplexbildner und den Kalzium-Ionen im Blut abgeschlossen ist. Dies wird dadurch erreicht, daß der Thrombintablette entsprechende Füllstoffe und/oder Sprengmittel zugesetzt werden.

Mit dem erfindungsgemäßen Test-Kit steht dem Großtierpraktiker erstmals ein einfacher, unkomplizierter semiquantitativer Kalzium-Schnelltest zur Verfügung, der vor Ort eine zuverlässige Kalzium-Grenzwertbestimmung ermöglicht.

Nur ein Teströhrchen bei der Anwendung bedeutet einfachere und sicherere Handhabung.

Die große Menge von 10 ml Blut als Testmenge bedeutet eine Verminderung der Gefahr von Falschaussagen des Testergebnisses durch Pipettier- oder Abfüllungsfehler. Nur ein Testwert verhindert Fehlreaktionen des Anwenders (falsche Blutmenge = veränderte Testaussage). Temperaturschwankungen beeinflussen die Reaktionszeit nur unwesentlich, das Testergebnis und somit die Testaussage stellt sich sicher innerhalb von spätestens 5 Minuten Reaktionszeit ein.

Die "Thrombintablette" löst sich zeitverzögert auf, so daß das auf die Blutgerinnung beschleunigend wirkende Thrombin erst jetzt langsam freikommt. Die Auflösung der Tablette ist nach ca. 2 Minuten beendet. Diese Zeit kann variieren. Die Form der Tablette ist unerheblich. Je nach Bedarf können unterschiedliche Füllstoffe, Sprengmittel und Preßdrücke bei der Tablettenherstellung verwendet werden.

Es ist auch möglich, eine "Kapsel" mit Thrombin im Inneren (als Pulver, pelletiert oder fest gepreßt) zu verwenden. Auch eine Filmtablette mit Überzug ist möglich. Bedingung für alle Zusätze in oder um die "Tablette" herum ist, daß sie keinerlei Einfluß auf das Testergebnis haben dürfen.

Durch eine Variation der EDTA-Einwaage und/oder der Einfüllmenge des Blutes kann der gewünschte Testwert der Anordnung eingestellt werden. Denkbar ist der Einsatz des vorgeschlagenen, erfindungsgemäßen, semiquantitativen Kalziumtests nicht nur beim Rind, sondern auch bei anderen Tierarten oder auch in der Humanmedizin.

Die nachfolgenden Beispiele und Vergleichstests sollen die Erfindung näher erläutern, ohne sie jedoch in ihrem Umfang zu beschränken.

Material:

- 1 Teströhrchen, aus Kunststoff gefertigt, mit flexiblem glattflächigem Kunststoffstöpsel und glattem Boden, der ein eigenständiges Stehen des Röhrchens ermöglicht. Der Querdurchmesser des Reagenzröhrchens erlaubt auch ein Aufbewahren in handelsüblichen Reagenzglasständern.
- In das Röhrchen sind 0,00431279 g Na2-EDTA eingedampft.
- Eine weitere Tablette ist zusätzlich in die Röhrchen verbracht worden, die 3 i.E. lyophilisiertes Thrombin, eingepreßt in ihrer Füllsubstanz, eine leicht lösliche Glucose, enthält.

Testausführung:

- 10 ml Vollblut werden blasenfrei mittels einer handelsüblichen Kunststoffeinmalspritze in das Teströhrchen eingefüllt. Das Röhrchen wird anschließend verstöpselt und 2 Minuten lang behutsam gekippt (nicht schütteln, damit kein Schaum entsteht), um den Inhalt gut zu durchmischen. Dabei löst sich das eingedampfte, flockige Na-EDTA zunächst innerhalb von Sekunden auf, vermischt sich mit dem Vollblut und komplexiert das, im Vollblut enthaltene Kalzium. Innerhalb der 2 Minuten Schwenkzeit löst sich zudem die Thrombintablette vollständig auf.

- Nun wird das Teströhrchen für die Testzeit von 5 Minuten ruhig hingestellt.

Funktionsprinzip des Ca-Tests:

Die $Na_2$-EDTA-Vorlage im Teströhrchen ist so berechnet, daß 0,466 mg Kalzium (das ist die absolute Menge Gesamt-Kalzium, die im Serum von 10 ml Vollblut enthalten ist, wenn der Hämatokrit 33,5 Vol.% und die Kalziumkonzentration im Serum 7 mg/dl beträgt) komplex abgebunden werden können.
Ist aber nun mehr als 7 mg/dl Kalzium in der Probe vorhanden, so bleiben noch freie Ca-Ionen in der Probe zurück, die mit Hilfe des, mit der Tablette zugefügten Thrombins als Reaktionsbeschleuniger die Blutgerinnung einleiten. Wurden dagegen alle Kalzium-Ionen mittels der EDTA-Vorlage abgebunden, kann die Blutgerinnung nicht mehr ablaufen.

Auswertung:

Die Ablesung erfolgt nach 5 Minuten nach folgendem, auch für die anderen Ca-Schnelltests gültigem Schema:
- geronnen = $Ca^{++}$-Wert über 7 mg/dl
- nicht geronnen = $Ca^{++}$-Wert unter 7 mg/dl
- nur Koagulationspunkte oder gallertige, noch fließfähige Beschaffenheit der Probe = Grenzwert 7 mg/dl.

Resultate:

Erste Resultate dieses modifizierten Ca-Tests stammen aus dem Jahr 1985. Zu diesem Zeitpunkt sah der Testablauf noch vor, das Thrombin nach dem Vermischen mit dem EDTA flüssig zuzugeben. Hierzu wurde das Thrombin als Topostasin® (Fa. La Roche) käuflich erworben. Hier liegt ein lyophilisiertes Pulver mit 3000 N.i.H. Einheiten vor, das dann auf eine solche Verdünnungsstufe gebracht wurde, daß 0,1 ml Flüssigkeit einer Aktivitätsmenge von 3 i.E. Thrombin entsprechen. Ansonsten wurde wie oben beschrieben verfahren.
Die Testergebnisse von 236 Untersuchungen an Kühen sind in Tabelle 7 dargestellt. Zur Kontrolle wurden die Kalziumwerte noch photometrisch bestimmt.

**Tab. 7: Vergleich der mit dem erfindungsgemäßen Test ermittelten und dem photometrisch bestimmten Serum-Kalziumgehalt bei 236 Kühen. Testgrenzwert ist 7 mg/dl.**

| | | Photometrische Ergebnisse | | |
|---|---|---|---|---|
| | | Ca über 7 mg/dl | Ca unter 7 mg/dl | |
| Erfindungs-gemäße Er-gebnisse | Ca über 7 mg/dl | 51 | 3 | 54 |
| | Ca unter 7 mg/dl | 17 | 165 | 182 |
| | | 68 | 168 | 236 |

| - Testempfindlichkeit: | 98 % | Von 168 hypokalzämischen Tieren wurden 165 richtig erkannt. |
|---|---|---|
| - Testspezifität: | 75 % | Von 68 nicht-hypokalzämischen Tieren wurden 51 richtig erkannt. |
| - Testrichtigkeit: | 92 % | 216 von insgesamt 236 Probanden wurden richtig klassifiziert. |
| pvT + | 91 % | 165 von 182 als hypokalzämisch ausgewiesenen Probanden waren tatsächlich hypokalzämisch. |
| pvT- | 94 % | 51 von 54 als normokalzämisch ausgewiesenen Probanden waren tatsächlich normokalzämisch. |
| - Prävalenz: | 71 % | Um die Aussagekraft des pvT + Wertes abschätzen zu können, ist es wichtig, die "Prävalenz" der an Hypokalzämie erkrankten Tiere in der Patientengruppe zu kennen. Hier sind es 168 von 236 Tieren! |

In der Tabelle 8 sind die Testergebnisse bisher bekannter Kalziumtests mit dem erfindungsgemäßen aufgeführt.

Diese Ergebnisse belegen, daß der erfindungsgemäße Test nicht nur einfach durchzuführen ist, sondern auch eine große Zuverlässigkeit aufweist. Besonders hervorzuheben ist eine Prävalenz von 71 %, da dieser Wert etwas über die Zuverlässigkeit der hypokalzämischen Bestimmung aussagt. Gerade die Hypokalzämie erfordert die schnelle und richtige Therapie des Großtierpraktikers.

Tab. 8

| Vergleich der verschiedenen Testergebnisse in % | | | | | | |
|---|---|---|---|---|---|---|
| | Test nach MAYER et al. | | | Test nach SANDHOLM (1979) | Test nach LINHART (1984) | Erfindungsgemäßer Ca-Test (Stand 1985) |
| | HARRY (1973) | MARTIG et al. | REITZ | | | |
| Empfindlichkeit | 22 | 95 | 81 | 89 | 75 | 98 |
| Spezifität | 52 | 100 | 63 | 94 | 95 | 75 |
| Richtigkeit | 48 | 98 | 70 | 91 | 87 | 92 |
| pvT + | 6 | 100 | 61 | 96 | 92 | 91 |
| pvT- | 82 | 97 | 81 | 84 | 84 | 94 |
| Prävalenz | 13 | 40 | 42 | 56 | 42 | 71 |

Die Bewertung hinsichtlich aller Testkriterien läßt sich noch verbessern, wenn die vorher beschriebenen Thrombintabletten zum Einsatz kommen, da nun eine gleichbleibende Aktivitätsmenge und eine größere Haltbarkeit des Reagens Thrombin erzielt wird. Dies kann an folgendem Beispiel kurz exemplarisch aufgezeigt werden:

| Ca-Wert (mg/dl) | Ca-Test-Graeub | Ca-Test erfindungsgemäß |
|---|---|---|
| 9,06 | * | * |
| 7,02 | * | gallertig |
| 7,0 | - | - |
| 6,8 | * | - |
| 6,6 | - | - |
| 4,62 | - | - |
| 4,5 | - | - |
| 4,1 | - | - |

* = geronnen - = nicht geronnen

Wie aus obiger Tabelle ersichtlich ist, ermöglicht der erfindungsgemäße Test eine bessere Bestimmung des vorgegebenen Grenzwertes von 7 mg Ca$^{++}$/dl.

**Patentansprüche**

1. Verfahren zur semiquantitativen Bestimmung des Kalziumgehaltes im Blut, bei dem die Kalzium-Ionen in einer Blutprobe entsprechend eines vorgegebenen Kalziumgrenzwertes mit einer definierten Menge eines Komplexbildners ganz oder teilweise komplexiert werden und anschließend die eventuell einsetzende Gerinnung durch einen Gerinnungsbeschleuniger aktiviert wird,

**dadurch gekennzeichnet,**
daß der Gerinnungsbeschleuniger Thrombin ist.

2. Verfahren nach Anspruch 1, dadurch gekennzeichnet, daß das Thrombin als Tablette zugesetzt wird.

3. Verfahren nach Anspruch 1 oder 2, dadurch gekennzeichnet, daß der Komplexbildner Natriumethylendiamintetraacetat (Na2-EDTA) ist.

4. Verfahren nach Anspruch 1 oder 2, dadurch gekennzeichnet, daß der Komplexbildner Kaliumethylendiamintetraacetat (K2-EDTA) oder eine ähnliche komplexbildende Substanz ist.

5. Verfahren nach einem der vorhergehenden Ansprüche, dadurch gekennzeichnet, daß zur Bestimmung eines Grenzwertes von 7 mg/dl (Kalzium/Blut) 4,313 mg Na2-EDTA und 2 bis 8 i.E. Thrombin verwendet werden.

6. Verfahren nach Anspruch 5, dadurch gekennzeichnet, daß 3 i.E. Thrombin verwendet werden.

7. Test-Kit zur semiquantitativen Serum-Kalziumbestimmung nach dem Verfahren eines der Ansprüche 1 bis 6, bestehend aus einem verschließbaren Probengefäß enthaltend eine definierte Menge eines Komplexbildners und eine Thrombintablette mit verzögerter Wirkstofffreisetzung.

8. Test-Kit nach Anspruch 7, dadurch gekennzeichnet, daß der Komplexbildner Natriumethylendiamintetraacetat (Na2-EDTA), Kaliumethylendiamintetraacetat (K2-EDTA) oder eine ähnliche komplexbildende Substanz ist.

9. Test-Kit nach Anspruch 7 oder 8, dadurch gekennzeichnet, daß es zur Bestimmung eines Grenzwertes von 7 mg/dl (Kalzium/Blut) 4,313 mg Na2-EDTA und 2 bis 8 i.E. Thrombin enthält.

10. Test-Kit nach Anspruch 9, dadurch gekennzeichnet, daß es 3 i.E. Thrombin enthält.

11. Verwendung einer Tablette, bestehend aus üblichen pharmazeutischen Füllstoffen und/oder Sprengmitteln und Thrombin, wobei die Thrombinfreisetzung verzögert ist, bei der Bestimmung des Blutkalziumgehalts nach dem Verfahren eines der Ansprüche 1 bis 6 oder bei einem Test-Kit nach einem der Ansprüche 7 bis 10.

12. Verwendung einer Kapsel mit in ihrem Inneren befindlichem Thrombin als pelletiertem oder fest gepreßtem Pulver, bei der Bestimmung des Blutkalziumgehalts nach dem Verfahren eines der Ansprüche 1 bis 6 oder bei einem Test-Kit nach einem der Ansprüche 7 bis 10.

13. Verwendung einer Filmtablette aus Thrombin mit einem Überzug bei der Bestimmung des Blutkalziumgehalts nach dem Verfahren eines der Ansprüche 1 bis 6 oder bei einem Test-Kit nach einem der Ansprüche 7 bis 10.

14. Verwendung einer Tablette, Kapsel und/oder Filmtablette nach einem der Ansprüche 11 bis 13, dadurch gekennzeichnet, daß die Auflösung der Tablette, Kapsel und/oder Filmtablette nach zwei Minuten beendet ist.

## Claims

1. Method for the semiquantitative determination of the calcium content in the blood, in which the calcium ions in a blood sample are in accordance with a preset calcium limit value complexed wholly or partly with a defined amount of a complexing agent, and subsequently any coagulation which occurs is activated by a coagulation accelerator,
characterised in that

the coagulation accelerator is thrombin.

2. Method according to Claim 1, characterised in that the thrombin is added as tablet.

3. Method according to Claim 1 or 2, characterised in that the complexing agent is sodium ethylenediaminetetraacetate ($Na_2$-EDTA).

4. Method according to Claim 1 or 2, characterised in that the complexing agent is potassium ethylenediaminetetraacetate ($K_2$-EDTA) or a similar complex-forming substance.

5. Method according to any of the preceding claims, characterised in that 4.313 mg of $Na_2$-EDTA and 2 to 8 i.u. of thrombin are used to determine a limit value of 7 mg/dl (calcium/blood).

6. Method according to Claim 5, characterised in that 3 i.u. of thrombin are used.

7. Assay kit for the semiquantitative determination of serum calcium by the method of one of Claims 1 to 6, consisting of a closeable sample vessel containing a defined amount of a complexing agent and a thrombin tablet with delayed release of active substance.

8. Assay kit according to Claim 7, characterised in that the complexing agent is sodium ethylenediaminetetraacetate ($Na_2$-EDTA), potassium ethylenediaminetetraacatate ($K_2$-EDTA) or a similar complex-forming substance.

9. Assay kit according to Claim 7 or 8, characterised in that it contains 4.313 mg of $Na_2$-EDTA and 2 to 8 i.u. of thrombin to determine a limit value of 7 mg/dl (calcium/blood).

10. Assay kit according to Claim 9, characterised in that it contains 3 i.u. of thrombin.

11. Use of a tablet consisting of conventional pharmaceutical fillers and/or disintegrants and thrombin, with the release of thrombin being delayed, in the determination of the blood calcium content by the method of one of Claims 1 to 6 or in an assay kit according to any of Claims 7 to 10.

12. Use of a capsule with thrombin present in its interior as pelleted or solidly compressed powder, in the determination of the blood calcium content by the method of one of Claims 1 to 6 or in an assay kit according to any of Claims 7 to 10.

13. Use of a film-coated tablet of thrombin with a coating in the determination of the blood calcium content by the method of one of Claims 1 to 6 or in an assay kit according to any of Claims 7 to 10.

14. Use of a tablet or a capsule and/or film-coated tablet according to any of Claims 11 to 13, characterised in that the dissolution of the tablet, capsule and/or film-coated tablet is complete after two minutes.

**Revendications**

1. Procédé pour la détermination semi-quantitative de la teneur en calcium dans le sang où les ions calcium, dans un échantillon de sang, sont totalement ou partiellement complexés, selon une valeur limite prédéterminée du calcium,avec une quantité définie d'un agent complexant et ensuite la coagulation qui débute éventuellement est activée par un accélérateur de coagulation,
   caractérisé en ce que
   l'accélérateur de coagulation est la thrombine.

2. Procédé selon la revendication 1, caractérisé en ce que la thrombine est ajoutée sous la forme de comprimés.

3. Procédé selon la revendication 1 ou 2, caractérisé en ce que l'agent complexant est l'acide sodium éthylènediaminetétraacétique (Na2-EDTA).

4. Procédé selon la revendication 1 ou 2, caractérisé en ce que l'agent complexant est l'acide potassium éthylènediaminetétraacétique (K2-EDTA) ou bien une substance complexante identique.

5. Procédé selon l'une quelconque des revendications précédentes, caractérisé en ce que, pour la détermination d'une valeur limite de 7 mg/dl (calcium/sang), on utilise 4,313 mg de Na2-EDTA et 2 8 U.I. de thrombine.

6. Procédé selon la revendication 5, caractérisé en ce qu'on utilise 3 U.I. de thrombine.

7. Trousse de test pour la détermination semiquantitative du calcium dans le sérum selon le procédé de l'une quelconque des revendications 1 à 6, se composant d'un récipient obturable pour l'échantillon contenant une quantité définie d'un agent complexant et un comprimé de thrombine avec libération retardée de l'agent actif.

8. Trousse de test selon la revendication 7, caractérisée en ce que l'agent complexant est l'acide sodium éthylènediaminetétraacétique (Na2-EDTA), l'acide potassium éthylènediaminetétraacétique (K2-EDTA) ou bien une substance complexante identique.

9. Trousse de test selon la revendication 7 ou 8, caractérisée en ce qu'elle contient pour la détermination d'une valeur limite de 7 mg/dl (calcium/sang), 4,313 mg de Na2-EDTA et à 8 U.I.de thrombine.

10. Trousse de test selon la revendication 9, caractérisée en ce qu'elle contient 3 U.I.de thrombine.

11. Utilisation d'un comprimé se composant de charges pharmaceutiques habituelles et/ou agents de dispersion et de thrombine, la libération de la thrombine étant retardée, dans la détermination de la teneur en calcium dans le sang selon le procédé de l'une quelconque des revendications 1 à 6 ou bien avec une trousse de test selon l'une quelconque des revendications 7 à 10.

12. Utilisation d'une capsule avec de la thrombine se trouvant à l'intérieur de celle-ci sous la forme d'une poudre solidement pressée ou en boulettes, dans la détermination de la teneur en calcium dans le sang selon le procédé de l'une quelconque des revendications 1 à 6 ou bien dans une trousse de test selon l'une quelconque des revendications 7 à 10.

13. Utilisation d'un comprimé en film de thrombine avec un recouvrement dans la détermination de la teneur en calcium dans le sang selon le procédé de l'une quelconque des revendications 1 à 6 ou bien dans le cas d'une trousse de test selon l'une quelconque des revendications 7 à 10.

14. Utilisation d'un comprimé, d'une capsule et/ou d'un comprimé en film selon l'une quelconque des revendications 11 à 13, caractérisée en ce que la dissolution du comprimé, de la capsule et/ou du comprimé en film est terminée au bout de deux minutes.